# DEMANDE DE BREVET EUROPEEN

(11) **EP 0 608 176 A1**
(43) Date de publication de la demande: **27.07.1994**
(21) Numéro de dépôt: 94400112.2
(22) Date de dépôt: 18.01.1994
(51) Int. Cl.: B05B 7/00, A61L 9/14

(54) **Micro-diffuseur pour brouillard de particules liquides**

(30) Priorité: 19.01.1993 FR 9300482
(71) Demandeur: BMB SARL, 64330 Garlin (FR)
(72) Inventeur: Accaries, Claude, F-64260 Arudy (FR)
(74) Mandataire: Ravina, Bernard

(57) **Abrégé**

Micro-diffuseur de très fines particules liquides dispersées, présentant une granulométrie pouvant être ajustée en deçà d'un seuil prédéterminé, comportant un mélangeur (2) dans lequel débouche d'une part un conduit (10) délivrant avec un débit donné un flux d'air sous pression, et d'autre part une tubulure d'amenée (21) d'un liquide à pulvériser, dispersé et entraîné dans le flux d'air en formant un brouillard admis à l'orifice d'entrée d'une chambre de fractionnement (22) munie à l'opposé d'un orifice de sortie (38) de ce brouillard dans l'atmosphère extérieure.

Selon l'invention, ce micro-diffuseur se caractérise en ce que la chambre de fractionnement comprend une pluralité de bagues creuses adjacentes (28), traversées par le flux d'air s'écoulant dans la chambre entre l'orifice d'entrée et l'orifice de sortie, ces bagues ménageant dans la chambre une série de chicanes successives à travers lesquelles le brouillard entraîné par le flux d'air subit autant de changements de direction, consécutivement à l'impact du flux dans chaque bague sur une surface en regard (33), appartenant à la bague et s'étendant transversalement vis-à-vis du sens d'écoulement de ce flux.

## Description

La présente invention est relative à un appareil micro-diffuseur, destiné notamment à la purification ou à la régénération d'ambiances, par dispersion dans un milieu environnant de micro-particules ou micro-gouttelettes dont les dimensions sont sensiblement homogènes entre elles et se situent en dessous d'une limite prédéterminée, en particulier de l'ordre de 1 à 2 microns au plus.

On connaît déjà des réalisations d'appareils de micro-diffusion, en particulier dans le domaine médical, pour des traitements de phytothérapie et plus spécialement d'aromathérapie, dans lesquels on procède à une dispersion très fine d'huiles ou essences diverses, notamment d'origine végétale, pour permettre leur respiration ou leur absorption par la peau ou les muqueuses d'un patient. Toutefois, ces appareils ont l'inconvénient de produire des particules relativement grandes et dont la dispersion n'est pas parfaite.

On sait par ailleurs que la régénération de certaines ambiances particulièrement polluées, notamment pour réaliser leur désodorisation, fait appel à la pulvérisation dans l'atmosphère correspondante de certains produits présentant une activité appropriée, permettant également et selon le cas de désinfecter ou de revitaliser l'air appauvri. Dans ces applications également, il est bien connu que la régénération de l'air est d'autant plus efficace que la susbstance pulvérisée est mieux dispersée et se présente sous la forme de particules très fines, aptes à former un brouillard ou un aérosol susceptible de se répartir uniformément et efficacement dans l'ensemble du volume d'air à traiter.

Les mêmes impératifs sont à satisfaire, pour des raisons sensiblement analogues, dans d'autres domaines où l'on fait aussi appel à une dispersion fine de gouttelettes liquides de très faible taille, notamment pour la pulvérisation en agriculture d'insecticides, herbicides ou fongicides, ou encore dans les injecteurs de moteurs à combustion, ou même dans les lances ou diffuseurs de produits contre l'incendie.

En outre, quelle que soit l'application envisagée, le contrôle de la taille des particules dispersées, notamment pour que celle-ci se situe en-deçà d'une seui 1 prédéterminé, permet de n'utiliser que les quantités strictement indispensables du produit diffusé pour satisfaire aux conditions de mise en oeuvre de l'application prévue, le produit utilisé pouvant dans certains cas être très coûteux, d'où l'intérêt immédiat d'éviter tout gaspillage ou perte inutile.

C'est le cas en particulier lorsque le produit à disperser est constitué par une huile aromatique, généralement désignée sous le terme d'huile essentielle, obtenue par extraction à partir de certains végétaux ayant des propriétés thérapeutiques, olfactives ou antiseptiques particulièrement développées et dont le coût est généralement élevé.

La présente invention concerne un appareil de micro-diffusion perfectionné qui allie à un ensemble en lui-même connu, apte à fournir un débit élevé d'air sous pression entraînant une quantité régulière et déterminée d'un produit liquide à disperser à la sortie de l'appareil dans l'environnement ambiant, des moyens permettant de fractionner les particules liquides entraînées pour leur donner une taille extrêmement réduite, sensiblement homogène dans l'ensemble du volume dispersé, en se situant en particulier en dessous d'une limite déterminée, ces moyens étant en outre agencés de manière à permettre d'agir sur le seuil de fractionnement adopté, en fonction de la nature du produit liquide et de l'application de l'appareil envisagée.

A cet effet et selon l'invention, le micro-diffuseur considéré, comportant un mélangeur dans lequel débouche d'une part un conduit délivrant avec un débit donné un flux d'air sous pression, et d'autre part une tubulure d'amenée d'un liquide à pulvériser, dispersé et entraîné dans le flux d'air en formant un brouillard admis à l'orifice d'entrée d'une chambre de fractionnement munie à l'opposé d'un orifice de sortie de ce brouillard dans l'atmosphère extérieure, dans lequel la chambre de fractionnement comprend une pluralité de moyens de fractionnement successifs traversés par le flux d'airs'écoulant dans la chambre entre l'orifice d'entrée et l'orifice de sortie, ces moyens ménageant dans la chambre une série de chicanes successives à travers lesquelles le brouillard entraîné par le flux d'air subit autant de changements de direction, se caractérise en ce que les moyens de fractionnement sont constitués par des bagues creuses adjacentes, comportant chacune une surface sur laquelle vient frapper le flux, cette surface s'étendant en travers du sens d'écoulement de celui-ci, chaque bague étant constituée d'un manchon tubulaire externe présentant un axe de symétrie, ouvert à une extrémité et comportant à l'extrémité opposée une pastille disposée transversalement par rapport au sens d'écoulement du flux, la pastille ayant une surface plus réduite que la section droite du manchon de manière à délimiter avec celui-ci un espace ouvert pour le passage du brouillard traversant la bague, consécutivement à l'impact du flux sur la pastille.

Dans un mode de réalisation préféré, la pastille de chaque bague est raccordée latéralement à la surface interne du manchon sur une fraction du pourtour de celui-ci, par une membrane de support pleine et étroite, de telle sorte que l'espace libre entre le manchon et la pastille s'étende sur la majeure partie au moins du manchon, en entourant la pastille.

Selon une caractéristique particulière de l'invention, la pastille comporte un contour circulaire, s'étend coaxialement au manchon et présente dans sa face sur lequel se produit l'impact du flux d'air un profil approximativement conique, de telle sorte que le brouillard entraîné par le flux d'air, après avoirfrap- pé la pastille, soit préférentiellement dévié et guidé latéralement en direction de l'espace libre délimité dans cette bague entre la pastille et le manchon.

Selon encore une autre caractéristique du micro-diffuseur considéré, les bagues adjacentes dans la chambre de fractionnement sont juxtaposées de telle sorte que les passages ménagés dans les bagues juxtaposées soient décalés d'une bague à la suivante selon la longueur de la chambre, dans le sens d'écoulement du flux d'air à l'intérieur de celle-ci.

De préférence, la chambre de fractionnement est constituée par un corps externe, délimitant une enceinte interne allongée, de forme générale cylindrique et de révolution autour d'un axe longitudinal, les bagues adjacentes étant logées dans la chambre en succession, chaque bague étant de préférence munie de moyens propres à permettre de modifier son orientation relative par rapport à l'axe longitudinal de la chambre selon trois directions respectivement perpendiculaires, en rotation, par déplacement axial et en inclinaison relative.

Selon une première variante d'exécution, le manchon de chaque bague est fileté extérieurement et coopère avec un filetage interne homologue, prévu dans la surface interne de l'enceinte de la chambre de fractionnement, pour permettre un déplacement de la bague selon la direction de l'axe de la chambre.

Selon une autre variante, la surface interne du corps de la chambre comprend un logement transversal pour le montage d'une vis à filet hélicoïdal, libre en rotation vis-à-vis du corps mais immobilisée en translation dans son logement, celui-ci étant ouvert en partie latéralement de manière à ce que la vis coopère avec une denture ménagée dans la surface externe du manchon d'une bague au moins afin de commander la rotation relative de cette bague autour de l'axe longitudinal de la chambre.

Dans encore une autre variante de réalisation, la paroi de la chambre comporte une rainure circulaire traversante, s'étendant sur une partie du contour d'une bague disposée en regard et à travers laquelle est engagée une fiche de manoeuvre, solidaire de cette bague de telle sorte que le déplacement de la fiche dans la rainure provoque la rotation de la bague par rapport à l'axe longitudinal de la chambre.

Dans un autre mode de réalisation, le manchon d'une au moins des bagues présente une surface partiellement sphérique et comporte extérieurement des tourillons axiaux engagés dans des trous formant paliers de support prévus dans la surface interne de la chambre, l'un au moins desdits tourillons comportant une fente de commande, accessible de l'extérieur du corps et permettant d'incliner la bague sur l'axe de la chambre.

Le micro-diffuseur selon l'invention permet ainsi de faire varier, au gré de l'utilisateur et selon la nature particulière de chacune des applications envisagées, le nombre et la disposition relative des bagues successives montées à l'intérieur de la chambre de fractionnement selon la longueur de celle-ci, en permettant ainsi de jouer sur les conditions dans lesquelles se produit l'i mpact du flux d'air entraînant le brouillard de particules ou gouttelettes liquides de telle sorte que, en faisant varier le nombre de ces points d'impact et les déviations du flux créées au droit des bagues par les pastilles appartenant à chacune de celles-ci, la dispersion des particules dans le brouillard délivré par l'orifice de sortie de la chambre puisse être ajustée à volonté, en réglant notamment la taille de ces particules de manière qu'elles présentent des dimensions voisines entre elles qui se situent de préférence en-deçà d'un seuil prédéterminé.

D'autres caractéristiques d'un micro-diffuseur réalisé conformément à l'invention apparaîtront encore à travers la description qui suit de plusieurs exemples de réalisation, donnés à titre indicatif et non limitatif, en référence aux dessins annexés sur lesquels :
- La Figure 1 est une vue en coupe longitudinale d'un micro-diffuseur réalisé conformément à un premier mode de réalisation de l'invention.
- Les Figures 2 et 3 sont des vues à plus grande échelle en perspective et en coupe partielle d'une bague mise en oeuvre dans la chambre de fractionnement du micro-diffuseur selon la Figure 1, cette bague étant respectivement représentée vue de dessus et de dessous afin d'illustrer de façon plus détaillée ses caractéristiques de forme et notamment le profil de la pastille centrale qu'elle comporte.
- Les Figures 4a et 4b sont des vues respectivement en coupe transversale et longitudinale, à légèrement plus grande échelle, d'une bague montée dans la chambre de fractionnement du micro-diffuseur et associée à des moyens permettant de faire varier l'orientation relative de cette bague par rapport à l'axe de la chambre.
- Les Figures 5a et 5b, 6a et 6b, 7a et 7b sont des vues analogues aux Figures 3a et 3b illustrant d'autres moyens propres à modifier l'orientation de chacune des bagues montées dans la chambre de fractionnement afin de faire varier respectivement sa position axiale, son orientation en azimuth et sa position angulaire par rapport à l'axe de la chambre de fractionnement.
- Les Figures 8 et 9 sont des vues plus schématiques en coupe axiale de deux autres variantes de réalisation du micro-diffuseur selon l'invention.
- La Figure 10 est une vue de détail d'une adaptation possible du mode de réalisation selon la Figure 7.
- La Figure 11 est une vue en coupe longitudinale d'une autre variante.

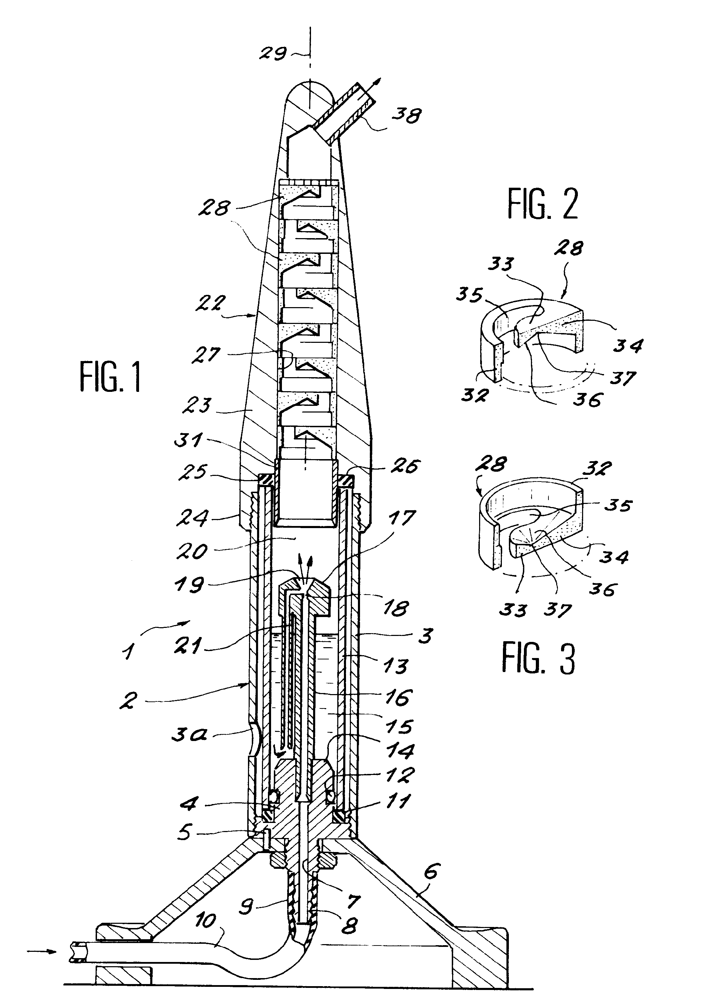

Sur la Figure 1, la référence 1 désigne de façon générale un appareil de micro-diffusion réalisé conformément à un premier mode de réalisation de l'invention.

Cet appareil comporte notamment un mélangeur 2, se présentant dans cet exemple sous la forme d'une enveloppe externe métallique 3, de forme générale cylindrique, fermée à son extrémité inférieure par un obturateur 4, l'ensemble étant assujetti par l'intermédiaire de pions de calage et d'immobilisation 5 sur un support 6, aménagé de manière à permettre de maintenir le mélangeur 2 en position sensiblement verticale, la forme et la structure particulières de ce support étant naturellement indifférentes à l'égard de la présente invention.

L'obturateur 4 comporte un alésage central 7 et se prolonge par un embout 8, propre à être raccordé à l'extrémité 9 d'une tuyauterie 10 délivrant dans l'alésage 7 et, au-delà, dans le mélangeur 2, un flux d'air avec un débit donné et sous une pression déterminée. Des joints, respectivement 11 et 12, assurent l'étanchéité du montage, notamment entre l'obturateur4 et un élément tubulaire 13, monté coaxialement à l'intérieur de l'enveloppe 3 en venant s'emboîter sur la partie centrale en saillie 14 l'obturateur 4. Avantageusement, cet élément tubulaire 13 est réalisé en un matériau transparent, notamment en verre du type "Pyrex" ou autre, et contient un volume donné d'un produit liquide 15 à pulvériser au moyen de l'appareil, en réalisant avec le flux d'air amené par la tuyauterie 10 un brouillard, formé par une dispersion homogène de particules ou gouttelettes de ce liquide, ces particules présentant une taille extrêmement réduite et dont la dimension peut être en outre ajustée au gré de l'utilisateur à l'aide des moyens qui seront décrits ci-après. Une fenêtre 3a est avantageusement prévue dans la paroi de l'enveloppe 3 afin de surveiller le niveau du liquide 15 dans l'élément tubulaire et transparent 13 du mélangeur.

De façon en elle-même classique dans la technique des appareils de pulvérisation, le mélangeur 2 comporte axialement une tubulure 16, se raccordant avec l'alésage 7 ménagé dans la partie centrale 14 de l'obturateur 4 et dans laquelle s'écoule le flux d'air comprimé délivré par la tuyauterie 10, cette tubulure se terminant, à son extrémité opposée par une tête 17, munie d'une buse 18 devant laquelle est ménagé un épanouissement 19 par lequel l'airs'échappe dans la partie supérieure 20 du mélangeur. Au niveau de la buse 18, débouche par ailleurs un conduit21 dirigé vers le bas et dont l'extrémité inférieure ouverte plonge dans le volume de liquide 15 contenu dans le mélangeur à l'intérieur de l'élément tubulaire 13, la dépression créée au droit de la buse 18 par l'écoulement du flux d'air sous pression et l'épanouissement 19 de la tête 17, agissant à la manière d'un Venturi, provoquant une aspiration continue du liquide à travers le conduit 21, suivie d'une dispersion de ce liquide dans la partie supérieure 20 à l'intérieur de l'enveloppe 3.

Sur cette enveloppe, est alors mise en place, conformément à l'invention, une chambre de fractionnement 22, comportant un corps 23, allongé, de forme générale cylindrique et prolongeant vers le haut le mélangeur 2, ce corps comprenant de préférence une collerette inférieure 24 se vissant sur une partie filetée prévue à l'extrémité correspondante de l'enveloppe 3. Un joint plat 25 est monté entre l'extrémité de l'élément tubulaire 13 et une portée d'appui 26 ménagée dans le corps 23 afin d'assurer l'étanchéité de la partie supérieure du mélangeur, de telle sorte que le brouillard délivré par la buse 18 soit totalement canalisé à l'intérieur de la chambre de fractionnement, dans le passage 27 ménagé axialement dans le corps 23, dans le prolongement de l'élément 13.

Selon l'invention, on dispose alors à l'intérieur du passage 27 une pluralité de bagues cylindriques 28, adjacentes et qui, selon le cas, peuvent être directement juxtaposées les unes aux autres, comme illustré sur la Figure 1, ou bien sontséparées d'une bague à la suivante par un espace approprié, permettant, comme décrit ci-après, de faire varier à la demande la position relative de ces bagues vis-à-vis de l'axe central de la chambre de fractionnement 22, cet axe étant schématisé sur le dessin sous la référence 29.

Dans le mode de réalisation représenté, les bagues 28 sont accolées et disposées sur toute la hauteur du passage 27, en étant immobilisées entre une portée d'appui 30 à la partie supérieure de ce passage et une bague de blocage 31 montée à l'extrémité supérieure de l'élément tubulaire 13.

Les Figures 2 et 3 illustrent de façon plus précise la structure particulière des bagues 28 mises en oeuvre dans le micro-diffuseur de la Figure 1. Chacune de ces bagues se présente de préférence sous la forme d'un manchon 32, de forme cylindrique et dont le diamètre externe correspond sensiblement au diamètre interne du passage 27, la bague comportant par ailleurs, en son centre, une pastille 33, raccordée à la paroi latérale du manchon 32 par une membrane 34 de liaison, conformée de telle sorte que la pastille 33 délimite entre son contour extérieur et la surface interne du manchon un espace libre 35, de forme circulaire et qui s'étend sur la majeure partie du manchon en entourant la pastille, à l'exception de la zone de liaison de celle-ci avec le manchon par la membrane 34.

Bien entendu, la largeur de l'espace 35 ainsi ménagé dans chaque bague 28 dépend du diamètre de la pastille centrale 33 et des conditions d'utilisation de la bague, telles que précisées ci-après. En outre, chaque bague et notamment le fond 36 de sa pastille 33 sont de préférence conformés de manière à présenter un profil sensiblement conique, dont le sommet 37 coïncide approximativement avec l'axe 29 du passage 27 lorsque la bague est montée dans ce dernier.

Le fonctionnement de l'appareil illustré sur la Figure 1 se déduit aisément de la description qui précède. La chambre de fractionnement 22, équipée de l'ensemble des bagues 28 superposées, montées comme représenté dans le passage 27, est fixée sur l'extrémité du mélangeur 2, par vissage sur ce dernier. Celui-ci est alors mis en route par l'envoi vers la buse 18 d'un flux d'air sous pression délivré par une source appropriée (non représentée) et admis par la tuyauterie 10 dans le mélangeur, provoquant simultanément l'aspiration d'une quantité appropriée de liquide à partir du volume 15 à travers le conduit 21, la buse 18 fournissant en continu dans la partie supérieure 20 de l'enveloppe tubulaire 13 un brouillard formé parla dispersion des particules ou gouttelettes de ce liquide dans le débit d'air.

Sous l'effet de la pression, ce brouillard pénètre dans la chambre de fractionnement 22 et se dirige vers la première bague 28 contre laquelle le flux chargé de liquide vient frapper, notamment dans la région de sa pastille centrale 33 en direction du sommet 37 du cône ménagé dans la face en regard de cette pastille, qui le dévie latéralement vers l'espace 35 par lequel il peut franchir la bague et venir rencontrer la bague 28 suivante, et ainsi de suite jusqu'à l'extrémité opposée de la chambre.

Le brouillard entraîné par le flux d'air subit, ainsi à la traversée de la chambre de fractionnement un trajet selon des chicanes successives avec, dans chacune des bagues, un changement de direction et un impactsurla pastille correspondante, permettant, comme l'expérience le démontre, de fractionner en particules ou gouttelettes de taille de plus en plus fine la fraction liquide entraînée, jusqu'à ce que cette dernière soit évacuée de la chambre par un bec de sortie 38 prévu à sa partie supérieure.

On constate notamment qu'avec un tel appareil, dans lequel le débit d'air à l'entrée est par exemple compris entre 150 et 200 I/h, de préférence de l'ordre de 170 I/h, avec un nombre de bagues entre 6 et 10 et de préférence de l'ordre de 8, on peut obtenir un brouillard à la sortie de l'appareil dans lequel les particules liquides pulvérisées présentent une granulométrie sensiblement constante à température ambiante, avec une dimension extrêmement homogène de ces particules de l'ordre, voire même inférieure à 2 microns, chaque bague assurant en quelque sorte un piégeage momentané des particules les plus grosses et un fractionnement de celles-ci, l'effet des diverses bagues se cumulant depuis l'entrée jusqu'à la sortie de la chambre. La granulométrie moyenne des particules dans le brouillard délivré peut être notamment contrôlée à l'aide d'un appareil classique du genre de celui connu sous le terme d'impacteurd'An- dersen ou encore au moyen d'un granulomètre à laser.

Le liquide pulvérisé peut être de toute nature appropriée mais est de préférence constitué par une huile dite "essentielle", à base d'essences végétales, présentant notamment un fort pouvoir de désinfection et/ou de purification de l'atmosphère ambiante. A titre indicatif, on peut ainsi utiliser de l'essence d'eucalyptus ou encore toute autre essence d'huile aromatique appropriée.

On a toutefois constaté que, avec certains autres produits pour des applications différentes et non pas seulement pour la production d'un brouillard désodorisant, par exemple pour la purification ou la désinfection de certaines atmosphères ou la réalisation d'injecteurs de carburant, le pouvoir de fractionnement du micro-diffuseur selon l'invention peut encore être amélioré si les diverses bagues 28 qui forment les chicanes de la chambre 22 à la sortie du mélangeur 2 peuvent présenter de l'une à l'autre une orientation variable ou encore être agencées de telle sorte que leur distance ou leur inclinaison relative puissent être modifiées et ajustées d'une bague à la suivante notamment.

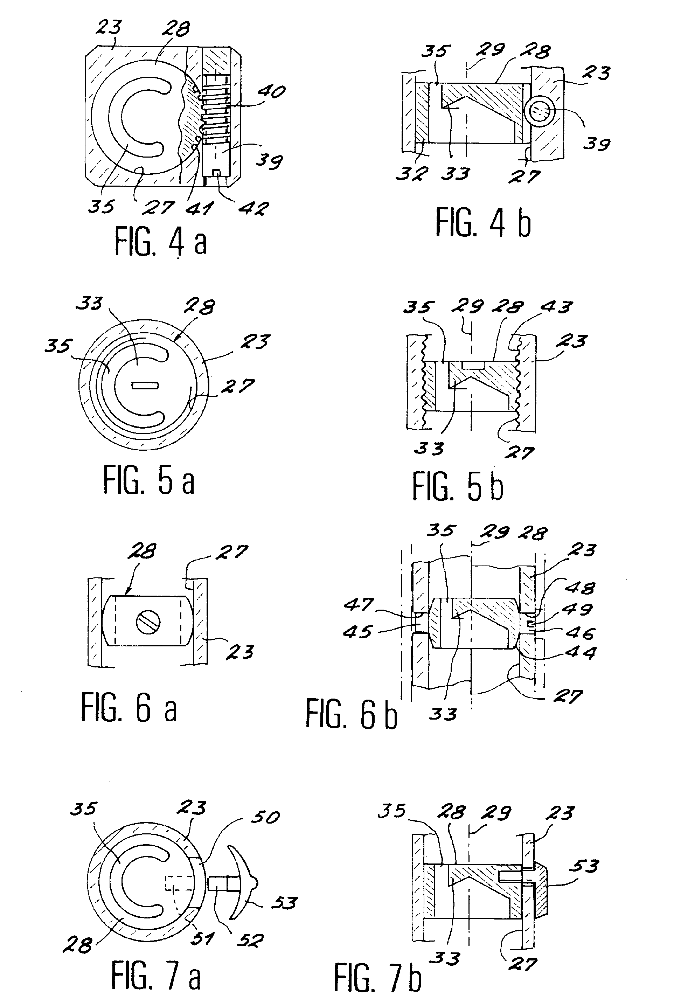

Les Figures 4 à 7 illustrent schématiquement divers modes de réalisation de mécanismes permettant ainsi d'agir sur les bagues individuellement pour faire varier leur présentation respective sur le trajet du brouillard entraîné par le flux d'air à la sortie du mélangeur.

A la Figure 4, on a ainsi envisagé de monter dans la paroi du corps 23 de la chambre 22 une vis transversale 39, comportant un filet hélicoidal 40 en prise avec une denture 41 prévue dans la surface externe du manchon 32 de la bague 28 correspondante, la rotation de cette vis au moyen d'un outil approprié coopérant avec une rainure 42 ménagée dans la tête de celle-ci, immobilisée en translation mais libre en rotation, permettant ainsi de faire tourner la bague sur elle-même autour de l'axe 29 de la chambre et parsui- i-te de modifier la position relative de l'espace libre 35 entourant la pastille 33 et par lequel s'échappe le brouillard lors de son entraînement, en passant de cette bague à la suivante.

Sur la Figure 5, illustrant un autre mode de réalisation, la paroi interne du passage 27 dans lequel sont disposées les bagues 28 comporte un filetage 43, permettant de la même manière d'orienter la position relative de chaque bague mais également de déplacer celle-ci selon la direction de l'axe 29, consécutivement à un nombre de tours appropriés de cette bague vis-à-vis du filetage.

Sur la variante selon la Figure 6, on a représenté une bague 28 dont la surface externe 44 présente un profil cylindrique, cette bague étant munie de deux tourillons, respectivement 45 et 46, pénétrant dans des trous ou alésages tels que 47 et 48 ménagés dans la paroi interne du corps 23 pour former des paliers de support de la bague, l'un des tourillons étant muni d'une rainure 49 accessible de l'extérieur de la chambre 22 et permettant d'orienter la bague, notamment en l'inclinant vis-à-vis de l'axe 29 dans le passage 27.

Sur la Figure 7 enfin, le corps 23 comporte au droit de la bague 28 une rainure transversale 50, la bague étant munie d'un logement borgne 51 dans lequel peut s'engager l'extrémité d'une fiche 52, susceptible de se déplacer à l'aide d'une bouton de commande 53 à l'intérieur de la rainure 50, en orientant la position de la bague 28 autour de l'axe 29.

Bien entendu, il va de soi que l'Homme de l'art pourra aisément envisager d'autres modes de réalisation pour les bagues montées dans le passage cylindrique de la chambre de fractionnement, afin d'assurer selon les besoins de l'application prévue une modification ou un changement commandé d'orientation de chacune des bagues, voire seulement d'une ou de plusieurs d'entre elles, afin d'ajuster leur orientation relative et l'effet de dispersion et de réduction en taille des particules liquides contenues dans le brouillar délivré par le mélangeur situé en amont de cette chambre.

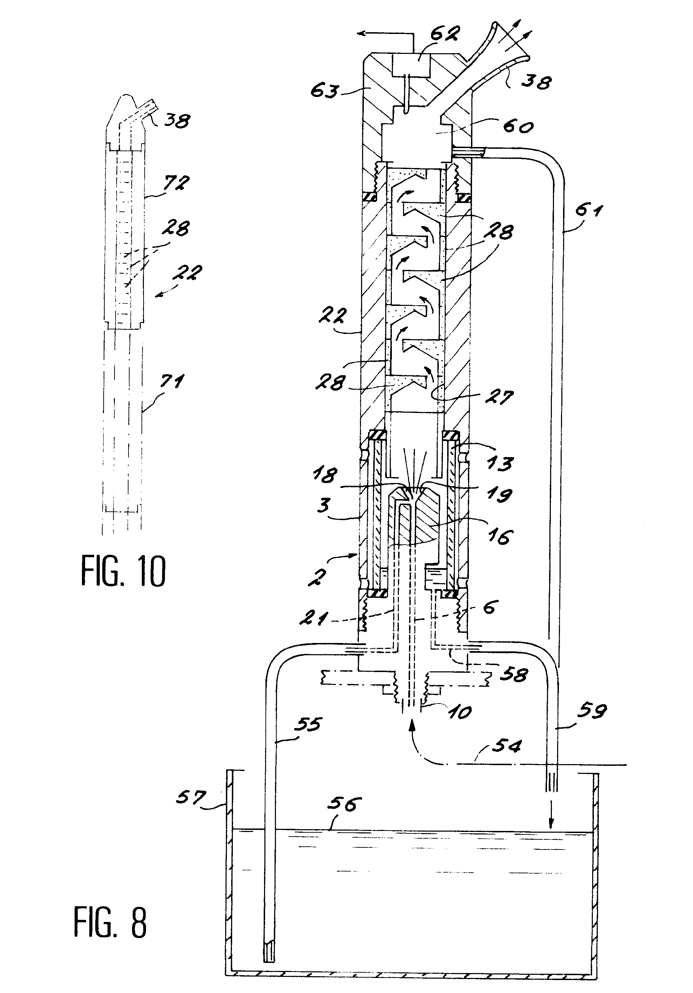

La Figure 8 illustre une autre variante de réalisation de l'appareil micro-diffuseur selon l'invention, dans lequel on retrouve néanmoins un mélangeur 2 et, à la sortie de celui-ci, une chambre de fractionnement 22. Sur cette Figure, on a en outre repris des chiffres de référence identiques pour désigner des organes semblables à ceux utilisés dans le premier mode de réalisation selon la Figure 1.

Dans cette variante, l'air comprimé acheminé par une conduite 54 se raccordant à la tuyauterie d'entrée 10 du mélangeur 2, assure la dispersion d'un produit liquide prélevé au moyen d'une tubulure 55 sous le niveau 56 d'une réserve de celui-ci contenue dans un bac 57 de grande dimension.

Le mélangeur, dont la structure est pour le reste très voisine de celle déjà décrite, comporte en plus des moyens permettant de récupérer les particules liquides qui, du fait de leur impact sur les bagues 28 dans la chambre de fractionnement 22, se séparent du flux d'air et retombent vers le mélangeur, le liquide recueilli dans le fond de ce dernier étant repris par une tubulure 58 et renvoyé par une conduite de trop-plein 59 vers le bac 57. De même, à la partie supérieure de la chambre 22, le liquide qui ruisselle en partie sur les parois de la chambre et qui n'est donc pas entraîné par le brouillard dans l'espace collecteur 60 ménagé au-delà de l'empilement des bagues et dans lequel débouche le bec de sortie 38, est récupéré par une seconde conduite de trop-plein 61 pour être de façon analogue renvoyé dans le bac 57, afin d'éviter toute perte ou gaspillage. Dans cet exemple, on peut avantageusement prévoir de monter des capteurs 62 dans le bouchon 63 qui ferme la partie supérieure de la chambre 22, ces capteurs permettant de contrôler en permanence la pression du brouillard délivré, et également de contrôler la granulométrie de ce dernier, par exemple au moyen d'un prélèvement fractionné ou continu, transmis à un appareil de mesure (non représenté).

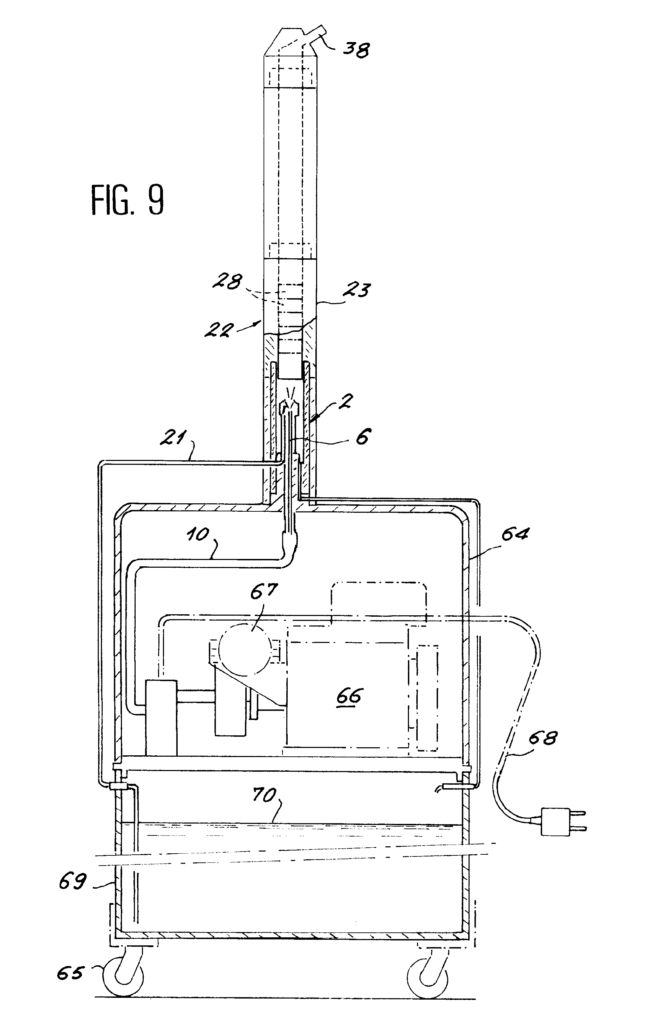

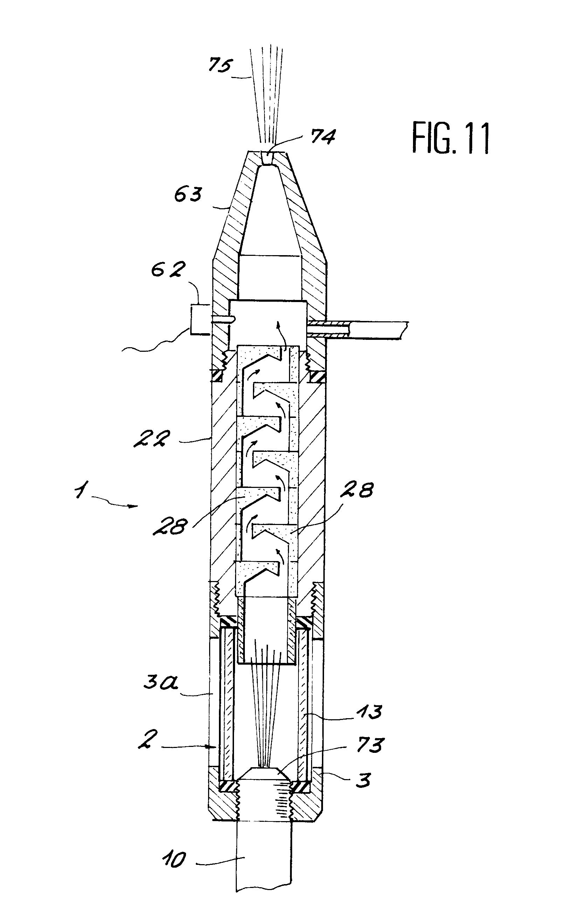

La Figure 9 illustre une autre variante d'un appareil transportable, comportant un carter 64 supporté par des roulettes 65 qui permettent de le déplacer sur le sol à volonté. Ce carter contient un compresseur 66, entraîné par un moteur électrique 67 mis sous tension au moyen d'un fil de connexion 68. En outre, la partie inférieure du carter 64 forme bac 69 pour le stockage d'un volume approprié du liquide 70 à pulvériser. Pour le reste, la structure de l'appareil est semblable à celle de ceux décrits dans ce qui précède. Toutefois, la chambre de fractionnement 22 peut être avantageusement conçue de telle sorte qu'elle soit elle-même formée de plusieurs parties successives telles que 71 et 72 (Figure 10), chacune de ces parties contenant un empilage de bagues 28 comme déjà décrit, cette solution permettant d'accroître encore le pouvoir de fractionnement et de dispersion du brouillard avant qu'il ne soit délivré vers l'environnement extérieur par le bec de sortie 38.

On réalise ainsi un micro-diffuseur de conception très simple qui permet de satisfaire à de très nombreuses applications, en particulier dans le domaine de la purification d'une atmosphère ambiante, de la désinfection de locaux, de la climatisation ou de la désodorisation. Comme déjà précisé, d'autres variantes sont bien entendu envisageables, en particulier dans le domaine médical pour certains traitements d'aromathérapie ou encore pour toutes applications où il s'agit de disposer d'un brouillard liquide très finement divisé et dans lequel les particules liquides dispersées ont une taille homogène, inférieure à une limite donnée.

Il donc de soi que l'invention ne se limite pas aux exemples plus spécialement décrits et représentés en référence aux dessins annexés ; elle en embrasse au contraire toutes les variantes couvertes par les revendications ci-après. Ainsi et comme illustré sur la Figure 11, le dispositif de microdiffusion, comportant dans la chambre de fractionnement 22 l'empilement des bagues 28, peut être utilisé pour pulvériser de manière optimale le fluide délivré par un brûleur de combustion, par exemple du genre d'un hydrocarbure, à usage industriel ou domestique.

Dans cette variante, la tuyauterie 10 qui amène le flux d'air au mélangeur 2, comporte une buse 73, qui délivre un mélange de ce fluide avec cet air selon un jet sous pression, ce dernier traversant ensuite la chambre de fractionnement 22 en franchissant les bagues 28 successives jusqu'à aboutir dans le bouchon de fermeture 63 prévu à l'extrémité opposée de cette chambre, ce bouchon étant muni en son centre d'un orifice calibré 74 à travers lequel le jet de fluide pulvérisé 75 est projeté à l'extérieur sous une forme extrêmement divisée, avec une granulométrie homogène des particules qu'il comporte.

Une telle solution permet d'obtenir une flamme efficace, brûlant dans des conditions optimales et n'exigeant qu'une consommation réduite.

## Revendications

1 - Micro-diffuseur de très fines particules liquides dispersées, présentant une granulométrie pouvant être ajustée en deçà d'un seuil prédéterminé, comportant un mélangeur (2) dans lequel débouche d'une part un conduit (10) délivrant avec un débit donné un flux d'air sous pression, et d'autre part une tubulure d'amenée (21) d'un liquide à pulvériser, dispersé et entraîné dans le flux d'air en formant un brouillard admis à l'orifice d'entrée d'une chambre de fractionnement (22) munie à l'opposé d'un orifice de sortie (38) de ce brouillard dans l'atmosphère extérieure, dans lequel la chambre comprend une pluralité de moyens de fractionnement successifs traversés par le flux d'airs'écoulant dans la chambre entre l'orifice d'entrée et l'orifice de sortie, ces moyens ménageant dans la chambre une série de chicanes successives à travers lesquelles le brouillard entraîné par le flux d'air subit autant de changements de direction, caractérisé en ce que les moyens de fractionnement sont constitués par des bagues creuses adjacentes (28), comportant chacune une surface sur laquelle vient frapper le flux, cette surface s'étendant en travers du sens d'écoulement de celui-ci, chaque bague étant constituée d'un manchon tubulaire externe (32) présentant un axe de symétrie, ouvert à une extrémité et comportant à l'extrémité opposée une pastille (33) disposée transversalement par rapport au sens d'écoulement du flux, la pastille ayant une surface plus réduite que la section droite du manchon de manière à délimiter avec celui-ci un espace ouvert (35) pour le passage du brouillard traversant la bague, consécutivement à l'impact du flux sur la pastille.

2 - Micro-diffuseur selon la revendication 1, caractérisé en ce que la pastille (33) de chaque bague (28) est raccordée latéralement à la surface interne du manchon (32) sur une fraction du pourtour de celui-ci, par une membrane de support (34) pleine et étroite, de telle sorte que l'espace libre (35) entre le manchon et la pastille s'étende sur la majeure partie au moins du manchon, en entourant la pastille.

3 - Micro-diffuseur selon l'une des revendications 1 ou 2, caractérisé en ce que la pastille (33) comporte un contour circulaire, s'étend coaxialement au manchon (32) et présente dans sa face sur lequel se produit l'impact du flux d'air un profil approximativement conique (36), de telle sorte que le brouillard entraîné par le flux d'air, après avoir frappé la pastille, soit préférentiellement dévié et guidé latéralement en direction de l'espace libre (35), délimité dans cette bague entre la pastille et le manchon.

4 - Micro-diffuseur selon l'une quelconque des revendications 1 à 3, caractérisé en ce que les bagues adjacentes (28) dans la chambre de fractionnement (22) sont juxtaposées de telle sorte que les passages libres (35) ménagés dans les bagues juxtaposées soient décalés d'une bague à la suivante selon la longueur de la chambre, dans le sens d'écoulement du flux d'air à l'intérieur de celle-ci.

5 - Micro-diffuseur selon l'une quelconque des revendications 1 à 4, caractérisé en ce que la chambre de fractionnement (22) est constituée par un corps externe (23), délimitant une enceinte interne (27), allongée, de forme générale cylindrique et de révolution autour d'un axe longitudinal (29), les bagues adjacentes (28) étant logées dans la chambre en succession, chaque bague étant de préférence munie de moyens propres à permettre de modifier son orientation relative par rapport à l'axe longitudinal de la chambre selon trois directions respectivement perpendiculaires, en rotation, par déplacement axial et en inclinaison relative.

6 - Micro-diffuseur selon la revendication 5, caractérisé en ce que le manchon (32) de chaque bague (28) est fileté extérieurement et coopère avec un filetage interne homologue (43), prévu dans la surface interne (27) de l'enceinte de la chambre de fractionnement (22), pour permettre un déplacement de la bague selon la direction de l'axe (29) de la chambre.

7 - Micro-diffuseur selon la revendication 5, caractérisé en ce que la surface interne (23) du corps de la chambre de fractionnement (22) comprend un logement transversal pour le montage d'une vis (39) à filet hélicoïdal (40), libre en rotation vis-à-vis du corps mais immobilisée en translation dans son logement, celui-ci étant ouvert en partie latéralement de manière à ce que la vis coopère avec une denture (41) ménagée dans la surface externe du manchon (32) d'une bague (28) au moins afin de commander la rotation relative de cette bague autour de l'axe longitudinal (29) de la chambre.

8 - Micro-diffuseur selon la revendication 5, caractérisé en ce que la paroi de la chambre de fractionnement (22) comporte une rainure circulaire (50) traversante, s'étendant sur une partie du contour d'une bague (28) disposée en regard et à travers laquelle est engagée une fiche de manoeuvre (52), solidaire de cette bague de telle sorte que le déplacement de la fiche dans la rainure provoque la rotation de la bague par rapport à l'axe longitudinal (29) de la chambre.

9 - Micro-diffuseur selon la revendication 5, caractérisé en ce que le manchon (32) d'une au moins des bagues (28) présente une surface partiellement sphérique et comporte extérieurement des tourillons axiaux (45, 46) engagés dans des trous (47, 48) formant paliers de support prévus dans la surface interne de la chambre de fractionnement (22), l'un au moins desdits tourillons (46) comportant une fente de commande (49), accessible de l'extérieur du corps et permettant d'incliner la bague sur l'axe de la chambre.

10 - Micro-diffuseur selon l'une quelconque des revendications 1 à 9, caractérisé en ce que la chambre de fractionnement (22) est constituée de plusieurs parties (71, 72) accolées.

11 - Micro-diffuseur selon l'une quelconque des revendications 1 à 10, caractérisé en ce qu'il comporte un carter mobile (64), comprenant un bac (69) formant réserve de liquide et un compresseur autonome (66) entraîné par un moteur (67), logés dans le carter.

12 - Micro-diffuseur selon l'une quelconque des revendications 1 à 11, caractérisé en ce que le conduit (10) comporte une buse (73) délivrant dans le mélangeur (2) un brouillard d'air et d'un fluide combustible, notamment du genre d'un hydrocarbure, l'orifice de sortie de la chambre de fractionnement (22) comprenant un orifice calibré pour l'évacuation de ce brouillard finement divisé.
